Europäisches Patentamt

European Patent Office

Office européen des brevets

(19)

(11) EP 1 250 889 A1

(12) EUROPEAN PATENT APPLICATION

(43) Date of publication:
23.10.2002 Bulletin 2002/43

(51) Int Cl.$^7$: A61B 8/06

(21) Application number: 01109277.2

(22) Date of filing: 17.04.2001

(84) Designated Contracting States:
AT BE CH CY DE DK ES FI FR GB GR IE IT LI LU
MC NL PT SE TR
Designated Extension States:
AL LT LV MK RO SI

(71) Applicants:
• FUKUDA DENSHI CO., LTD.
Tokyo 113-8483 (JP)
• Tei, Chuwa
Kagoshima, Kagoshima 891-0107 (JP)

(72) Inventors:
• Tei, Chuwa
Kagoshima, Kagoshima 891-0107 (JP)
• Tsubone, Izumi
Bunkyo-ku, Tokyo 113-8483 (JP)

(74) Representative: Seeger, Wolfgang, Dipl.-Phys.
SEEGER & SEEGER
Patentanwälte & European Patent Attorneys
Georg-Hager-Strasse 40
81369 München (DE)

(54) **Ultrasonic diagnostic device and method of measuring index for indicating vascular status**

(57) An ultrasonic diagnostic device which is capable of introducing an index for evaluating a vascular status which may be a burden on heart in the evaluation of cardiac functions and displaying the index, and a method of measuring an index for indicating a vascular status are provided.

The ultrasonic diagnostic device 10 comprises and is equipped with the blood flow measurement section 11 for measuring blood flow using Doppler effect of ultrasound, the blood pressure measurement section 12 for measuring blood pressure and the calculation section 13 for calculating an index Ntei for indicating a vascular status using a cardiac output Co obtained from a Doppler flow rate distribution measurement by means of the blood flow measurement section 11 and a value of blood pressure Bp obtained from a blood pressure measurement by means of the blood pressure measurement section 12 by the expression of Ntei = Co / Bp.

10: ULTRASONIC DIAGNOSTIC DEVICE

| BLOOD FLOW MEASUREMENT SECTION | — 11 |

Co

| CALCULATION SECTION
Ntei=Co/Bp | — 13 |

Bp

| BLOOD PRESSURE MEASUREMENT SECTION | — 12 |

Fig. 1

EP 1 250 889 A1

## Description

BACKGROUND OF THE INVENTION

Field of the Invention

The present invention relates to an ultrasonic diagnostic device and a method of measuring an index for indicating a vascular status.

Description of the related art

In recent years, in an ultrasonic diagnostic device for medical care, ultrasound is irradiated within a subject, a state of blood flow has been displayed by measurement using Doppler effect and a volume of blood flow has been measured.

By measurement of a volume of blood flow using such an ultrasonic diagnostic device, cardiac output Co which is sent out from heart to the entire system of the body is calculated, and employed as a critical value on the diagnosis.

In diagnosis, at present, a blood vessel status is estimated by a common blood pressure test, and the prescription of high blood pressure is determined.

However, since the blood pressure is determined by cardiac output Co and a blood vessel status of the subject, in a high blood pressure, even if the vascular system is normal, the value of cardiac output Co may be high.

Moreover, even in the case where the vascular system is hardened, when the value of cardiac output Co is low, the value of normal blood pressure is indicated.

SUMMARY OF THE INVENTION

The present invention has been carried out in consideration of the above-described conventional problems and the circumstances and an object of the present invention is to provide an ultrasonic diagnostic device which gives a more effective index for the purpose of precisely making a diagnosis and a method of measuring an index for indicating a vascular status.

Specifically, an ultrasonic diagnostic device of the present invention, wherein in an ultrasonic diagnostic device having at least a blood flow measurement section using Doppler effect of ultrasound and an automated blood pressure measurement section, the present invention is equipped with a calculation means for calculating an index for indicating a vascular status using cardiac output obtained from a measurement of a Doppler flow rate distribution by the foregoing blood flow measurement section and a value of blood pressure obtained from an automated blood pressure measurement by the foregoing automated blood pressure measurement section.

Now, in an ultrasonic diagnostic device of the above-described present invention, the above-described calculation section is preferably a section for calculating an index for indicating a vascular status based on the following expression:

$$Ntei = Co / Bp$$

where Ntei denotes an index for indicating a vascular status ; Co denotes a cardiac output; and Bp denotes a value of blood pressure.

Or, it will be also available that the other index such as index log (Co / Bp), index Bp / Co or the like is calculated instead of the above-described index Ntei = Co / Bp.

Moreover, in an ultrasonic diagnostic device of the above-described present invention, it is also preferable that an ultrasonic diagnostic device is equipped with an image display section for displaying an index for indicating the above-described vascular status.

Furthermore, in an ultrasonic diagnostic device of the above-described present invention, it is also preferable that an ultrasonic diagnostic device is provided with a blood flow measurement section and an automated blood pressure section separately.

Moreover, a method of measuring an index for indicating a vascular status of the present invention is characterized in that the method measures a value of blood pressure as well as find a cardiac output by measuring a Doppler flow rate distribution using Doppler effect of ultrasound, and calculates an index for indicating a vascular status using these cardiac output and value of blood pressure.

Now, in the method of measuring an index for indicating a vascular status of the present invention, preferably the index is calculated based on the following expression when the index for indicating vascular status thereof is found:

$$Ntei = Co / Bp$$

where Ntei denotes an index for indicating a vascular status ; Co denotes a cardiac output; and Bp denotes a value of blood pressure.

In an ultrasonic diagnostic device having at least a blood flow measurement section for measuring a blood flow by utilizing Doppler effect of ultrasound and a blood pressure measurement section for measuring a blood pressure, the ultrasonic diagnostic device of the present invention is equipped with a calculation section for calculating an index for indicating a vascular status using a cardiac output obtained from a measurement of a Doppler flow rate distribution by the foregoing blood flow measurement section and a value of blood pressure obtained from a blood pressure measurement by the foregoing blood pressure measurement section, thereby being capable of introducing an index for evaluating a vascular status which may be a burden on heart in the evaluation of cardiac functions and briefly examining a more proper status of circulation in a shorter time.

Moreover, if the expression of Ntei = Co / Bp is employed as an index for indicating this vascular status, the vascular status can be directly expressed.

Moreover, in an ultrasonic diagnostic device of the above-described present invention, in the case where the device is equipped with an image display section for displaying an index for indicating the above-described vascular status, a more proper circulation status can be grasped with the image by the ultrasonic diagnostic device itself and a course of treatment to the patient can be precisely determined.

Furthermore, in the case where a blood flow measurement section and a blood pressure measurement section are separately provided, the above-described object is achieved by remodeling the existing ultrasonic diagnostic device and blood pressure gauge, and the development cost can be made inexpensive and the reduced cost is realized comparing to the cost of the case where a new device is developed from the beginning.

Moreover, a method of measuring an index for indicating a vascular status of the present invention can introduce an index for evaluating a vascular status which may be a burden on heart in the evaluation of cardiac functions by calculating an index for indicating a vascular status on the basis of, for example, the expression of Ntei = Co / Bp using a cardiac output obtained from a Doppler flow rate distribution measurement and a value of blood pressure obtained from blood pressure measurement.

If this index is employed, a new direction can be indicated in therapeutic prescriptions, and it can be widely popularized as a simplified examination method which is capable of finding in an earlier stage an abnormal vascular status of a person who has been regarded as normal by the conventional measurement of the blood pressure.

BRIEF DESCRIPTION OF THE DRAWINGS

Fig. 1 is a functional block diagram of an ultrasonic diagnostic device of the present invention;
Fig. 2 is a schematic block diagram of an ultrasonic diagnostic device according to the embodiment of the present invention;
Fig. 3 is a diagram for illustrating a connection between a main body of an ultrasonic diagnostic device and an automated blood pressure gauge.
Fig. 4 is a diagram showing a configuration of a display screen of a monitor display; and
Fig. 5 is a drawing showing a display screen of a monitor display.

DESCRIPTION OF THE PREFERRED EMBODIMENTS

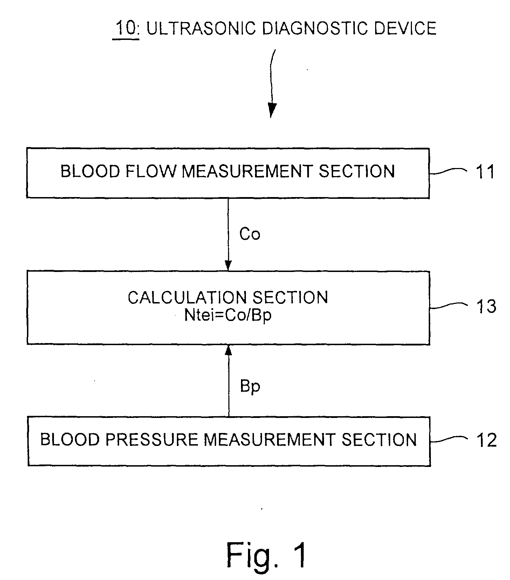

Hereinafter, one embodiment of the present invention will be described with reference to Fig. 1.

Fig. 1 is a functional block diagram of an ultrasonic diagnostic device of the present invention.

The ultrasonic diagnostic device 10 comprises and is equipped with the blood flow measurement section 11 for measuring blood flow using Doppler effect of ultrasound, the blood pressure measurement section 12 and the calculation section 13 for calculating an index Ntei for indicating a vascular status by the expression of Ntei = Co / Bp using a cardiac output Co obtained from a Doppler flow rate distribution measurement by the blood flow measurement section 11 and a value of blood pressure Bp obtained from a blood pressure measurement by the blood pressure measurement section 12.

Although the embodiment of the present invention will be described below in further detail, the scope of the present invention is not limited to this embodiment.

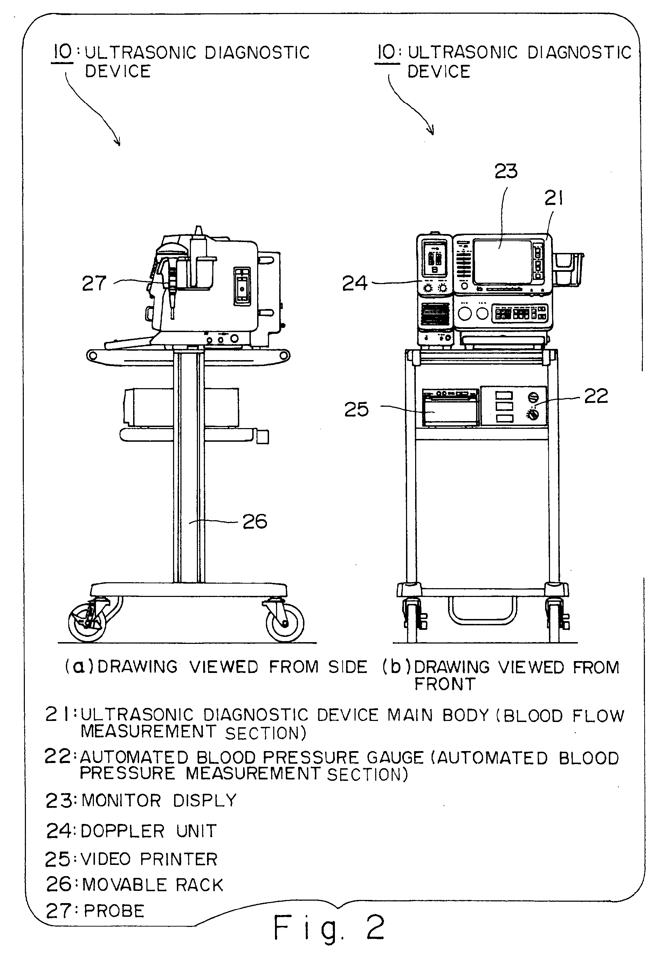

In Fig. 2, this ultrasonic diagnostic device 10 comprises the probe 27 for sending and receiving an ultrasound within the living body, the ultrasonic diagnostic device main body (blood flow measurement section) 21 having an image formation unit for forming an image based on a sending / receiving circuit, a scanning circuit and scanned echo data, the automated blood pressure gauge (automated blood pressure section) 22, the video printer 25 and the movable rack 26 for setting these bodies of equipments, and has the monitor display (image display section) 23 for displaying an index Ntei for indicating a vascular status by the expression of Ntei = Co / Bp using a cardiac output Co obtained from a Doppler flow rate distribution measurement by the blood flow measurement section 21 and avalue of blood pressure Bp obtained from a blood pressure measurement by the blood pressure measurement section 22, and the Doppler unit 24 for performing a Doppler analysis.

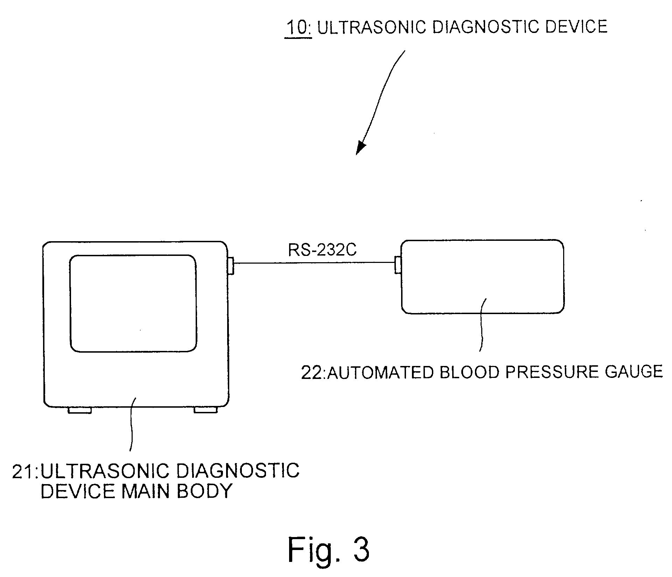

Referring to the connection between the ultrasonic diagnostic device main body 21 and the automated blood pressure gauge 22 as shown in Fig. 3, the ultrasonic diagnostic device main body (UF-5800A manufactured by Fukuda Denshi Co., Ltd.) 21 and the automated blood pressure gauge (BP103iII manufactured by Colin, Co., Ltd.) are connected by a serial interface of RS-232C.

Next, the calculation procedure of an index Ntei for indicating a vascular status measured by employing an ultrasonic diagnostic device of the present embodiment of the present invention will be described below.

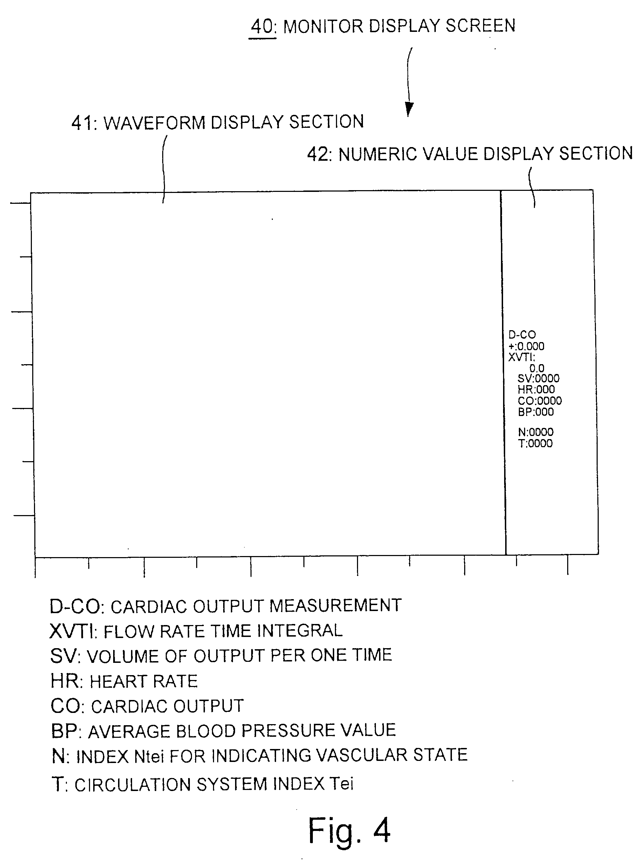

Fig. 4 is a diagram showing a calculation result display format of an index Ntei for indicating avascular status displayed on the screen of the monitor display (image display section) 23.

In Fig. 4, the screen constitution is made so that the left side of the monitor display screen 40 is occupied with the waveform display section 41 and the right side is occupied with the numeric value display section 42, and measured numeric values and calculated results are displayed on the numeric value display section 42. Out of units of numeric values shown here, that is, in Fig. 4, D-Co denotes cardiac output measurement mode, XVTI denotes flow rate time integral, SV denotes volume of output per one time, HR denotes heart rate, CO denotes cardiac output, BP denotes value of average blood pressure, N denotes index Ntei for indicating a vascular status and T denotes index Tei for circulation organ.

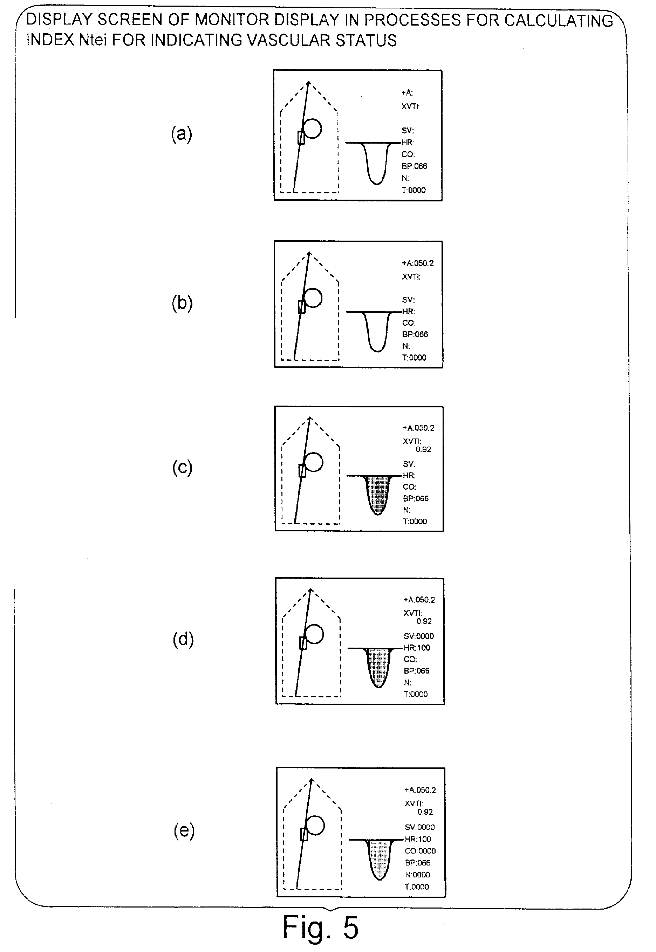

Next, the processes of calculating an index Ntei for indicating a vascular status will be described below (see Fig. 5 as appropriate).

Process 1: turn on the electric sources of the ultrasonic diagnostic device main body and the automated blood pressure gauge,

Process 2: carry out blood pressure measurement by the automated blood pressure gauge.

Process 3: the processes of measurement of ultrasonic diagnostic device (calculation of index Ntei for indicating a vascular status),

(1) Select the measurement item [D-CO] of the ultrasonic diagnostic device;
(2) Send out a request signal of the measured value of blood pressure from the ultrasonic diagnostic device main body to the automated blood pressure gauge;
(3) The ultrasonic diagnostic device main body receives the measured value of the blood pressure from the automated blood pressure gauge,
(4) The ultrasonic diagnostic device main body displays the received value of blood pressure,
(5) Display an index Ntei calculation result on the blank space for result of measurement item [D-CO] of the ultrasonic diagnostic device main body (Fig. 5 (a));
(6) Measure a sectional area of aorta (cm$^2$) by the ultrasonic diagnostic device main body (a sectional area of aorta is found by designating the diameter of aorta (distance between one end and the other end of blood vessel of aorta) displayed on the screen, for example, using + cursor of Fig. 5 (b) within the ultrasonic diagnostic device main body);
(7) Measure time flow rate integral (VTI) (m) by the ultrasonic diagnostic device main body (VTI is found in the ultrasonic diagnostic device main body by designating integral interval of time changing curve of flow rate displayed on the screen, for example, using +cursor of Fig. 5 (c));
(8) Measure manually or automatically HR (beat / min) by the ultrasonic diagnostic device main body (Fig. 5 (d));
(9) Display the result of the measurement item [D-CO] of the ultrasonic diagnostic device main body; and
(10) Display the index Ntei calculation result in the blank space for the result of the measurement item [D-CO] of the ultrasonic diagnostic device main body (Fig. 5 (e)).

Process 4: end (return to the process 2 and repeat the measurement according to the necessity).

## Claims

1. An ultrasonic diagnostic device, wherein in said ultrasonic diagnostic device having at least a blood flow measurement section for measuring blood flow using Doppler effect of ultrasound and a blood pressure measurement section for measuring blood pressure, said ultrasonic diagnostic device has a calculation section for calculating an index for indicating a vascular status using a cardiac output obtained from Doppler flow rate distribution measurement by said blood flow measurement section and a value of blood pressure obtained from blood pressure measurement by said blood pressure measurement.

2. The ultrasonic diagnostic device according to claim 1, wherein said calculation section calculates an index for indicating a vascular status based on the following expression:

$$Ntei = Co / Bp$$

where Ntei denotes an index for indicating a vascular status ; Co denotes a cardiac output; and Bp denotes a value of blood pressure.

3. The ultrasonic diagnostic device according to claim 1, wherein said ultrasonic diagnostic device has an image display section for displaying an index for indicating a vascular status which is calculated by said calculation section.

4. The ultrasonic diagnostic device according to claim 1, wherein said blood flow measurement section and said blood pressure measurement section are separately provided.

5. A method of measuring an index for indicating a vascular status, wherein a value of blood pressure is measured as well as a cardiac output is determined by measuring Doppler flow rate distribution using Doppler effect of ultrasound and an index for indicating a vascular status is calculated using these cardiac output and value of blood pressure.

6. The method of measuring an index for indicating a vascular status according to claim 1, wherein said relevant index is calculated based on the following expression upon determining the relevant index for indicating said vascular status:

$$Ntei = Co / Bp$$

where Ntei denotes an index for indicating a vascular status ; Co denotes a cardiac output; and Bp denotes a value of blood pressure.

10: ULTRASONIC DIAGNOSTIC DEVICE

| BLOOD FLOW MEASUREMENT SECTION | 11 |
|---|---|

Co

| CALCULATION SECTION<br>Ntei=Co/Bp | 13 |
|---|---|

Bp

| BLOOD PRESSURE MEASUREMENT SECTION | 12 |
|---|---|

# Fig. 1

10:ULTRASONIC DIAGNOSTIC DEVICE

10:ULTRASONIC DIAGNOSTIC DEVICE

27

23

21

24

25

22

26

(a)DRAWING VIEWED FROM SIDE  (b)DRAWING VIEWED FROM FRONT

21:ULTRASONIC DIAGNOSTIC DEVICE MAIN BODY (BLOOD FLOW MEASUREMENT SECTION)

22:AUTOMATED BLOOD PRESSURE GAUGE (AUTOMATED BLOOD PRESSURE MEASUREMENT SECTION)

23:MONITOR DISPLY

24:DOPPLER UNIT

25:VIDEO PRINTER

26:MOVABLE RACK

27:PROBE

Fig. 2

10: ULTRASONIC DIAGNOSTIC DEVICE

RS-232C

22:AUTOMATED BLOOD PRESSURE GAUGE

21:ULTRASONIC DIAGNOSTIC
DEVICE MAIN BODY

Fig. 3

<u>40</u>: MONITOR DISPLAY SCREEN

41: WAVEFORM DISPLAY SECTION

42: NUMERIC VALUE DISPLAY SECTION

D-CO
+:0.000
XVTI:
    0.0
SV:0000
HR:000
CO:0000
BP:000

N:0000
T:0000

D-CO: CARDIAC OUTPUT MEASUREMENT

XVTI: FLOW RATE TIME INTEGRAL

SV: VOLUME OF OUTPUT PER ONE TIME

HR: HEART RATE

CO: CARDIAC OUTPUT

BP: AVERAGE BLOOD PRESSURE VALUE

N: INDEX Ntei FOR INDICATING VASCULAR STATE

T: CIRCULATION SYSTEM INDEX Tei

# Fig. 4

DISPLAY SCREEN OF MONITOR DISPLAY IN PROCESSES FOR CALCULATING INDEX Ntei FOR INDICATING VASCULAR STATUS

Fig. 5

**European Patent Office**

**EUROPEAN SEARCH REPORT**

Application Number

EP 01 10 9277

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 4 796 634 A (TARBOX GARY L ET AL) 10 January 1989 (1989-01-10) * column 2, line 46 - line 66 * * column 21, line 36 - line 41 * * column 29, line 63 - line 68 * * column 30, line 1 - line 17 * | 1-6 | A61B8/06 |
| X | EP 0 330 463 A (COLIN ELECTRONICS) 30 August 1989 (1989-08-30) * column 3, line 52 - column 4, line 27 * * column 5, line 25 - line 45 * * column 5, line 61 - line 65 * * column 6, line 38 - line 48 * | 1-6 | |
| | | | TECHNICAL FIELDS SEARCHED (Int.Cl.7) A61B |

The present search report has been drawn up for all claims

| Place of search | Date of completion of the search | Examiner |
|---|---|---|
| THE HAGUE | 11 September 2001 | Knüpling, M |

EPO FORM 1503 03.82 (P04C01)

EP 1 250 889 A1

**ANNEX TO THE EUROPEAN SEARCH REPORT
ON EUROPEAN PATENT APPLICATION NO.**                    EP 01 10 9277

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2001

| Patent document cited in search report | | Publication date | Patent family member(s) | | Publication date |
|---|---|---|---|---|---|
| US 4796634 | A | 10-01-1989 | NONE | | |
| EP 0330463 | A | 30-08-1989 | JP | 1214335 A | 28-08-1989 |
| | | | JP | 2882797 B2 | 12-04-1999 |
| | | | EP | 0330463 A1 | 30-08-1989 |

EPO FORM P0459

For more details about this annex : see Official Journal of the European Patent Office, No. 12/82

12